# EUROPEAN PATENT APPLICATION

(11) **EP 4 660 186 A1**
(43) Date of publication of application: **10.12.2025**
(21) Application number: 25759263.4
(22) Date of filing: 07.03.2025
(51) Int. Cl.: C07C 253/34, C07C 231/06, C07C 237/30, C07C 255/58, C07C 309/30

(54) **ACID ADDITION SALT OF 4-AMINO-2-HALOBENZONITRILE COMPOUND AND METHOD FOR PRODUCING SAME**

(30) Priority: 25.03.2024 JP 2024048804
(71) Applicant: SUGAI CHEMICAL INDUSTRY CO., LTD., Wakayama-shi, Wakayama 6410043 (JP)
(72) Inventor: NISHINO Mitsuhiro, Wakayama-shi, Wakayama 6410043 (JP); OTSUKI Serika, Wakayama-shi, Wakayama 6410043 (JP)
(74) Representative: Grünecker Patent- und Rechtsanwälte PartG mbB
(86) International application number: PCT/JP2025/008471
(87) International publication number: WO 2025/204756

(57) **Abstract**

Provided is a process for industrially advantageously producing an acid addition salt of a 4-amino-2-halobenzonitrile compound with an organic sulfonic acid.

An acid addition salt of a 4-amino-2-halobenzonitrile compound represented by the following formula (2) with an organic sulfonic acid is produced. The production process comprises a separation step of separating an acid addition salt of a compound represented by the formula (2) with an organic sulfonic acid, from an acid addition salt of an isomeric mixture of a compound, represented by the following formula (1) and at least containing the compound represented by the formula (2), with an organic sulfonic acid, using a difference in solubility between acid addition salts of isomers in a solvent: 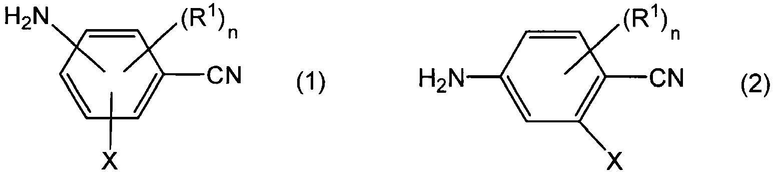 wherein X represents a halogen atom, R¹ represents a reactively inactive substituent, and n denotes an integer of 0 to 3.

## Description

### TECHNICAL FIELD

The present invention relates to an acid addition salt of a 4-amino-2-halobenzonitrile compound with an organic sulfonic acid (an organic sulfonic acid salt of a 4-amino-2-halobenzonitrile compound), and a process for producing the same; the acid addition salt is useful as a pharmaceutical intermediate, a reagent, and the like.

### BACKGROUND ART

4-Amino-2-fluorobenzamide, which is an aromatic compound having an amino group, a fluorine atom, and an amide group as substituents, is known to be useful as a pharmaceutical intermediate and the like.

As a process for producing 4-amino-2-fluorobenzamide, Chinese Patent Application Publication No. 116332789 specification (CN 116332789 A, Patent Document 1) discloses a process which comprises an amination step of converting one fluorine atom of 2,4-difluorobenzonitrile as a raw material to an amino group, a separation step of separating an isomer of the resulting amino-substituted compound, and an amidation step of converting a cyano group of the resulting 4-amino-2-fluorobenzonitrile to an amide group. As the separation step, disclosed is a distillation method or a method of forming an acid addition salt of an isomeric mixture of the amino-substituted compound with an acid and then separating an acid addition salt of 4-amino-2-fluorobenzonitrile with the acid by precipitation.

### CITATION LIST

### PATENT LITERATURE

Patent Document 1: CN 116332789 A

### SUMMARY OF INVENTION

### TECHNICAL PROBLEM

However, Examples of Patent Document 1 use a halogen-containing solvent as a solvent and a gaseous hydrochloric acid (a hydrogen chloride gas) difficult to handle, and are not industrially advantageous.

It is therefore an object of the present invention to provide a process for industrially advantageously producing an acid addition salt of a 4-amino-2-halobenzonitrile compound with an organic sulfonic acid (an organic sulfonic acid salt of a 4-amino-2-halobenzonitrile compound).

Another object of the present invention is to provide a process for easily or efficiently producing an acid addition salt of a 4-amino-2-halobenzonitrile compound with an organic sulfonic acid.

Still another object of the present invention is to provide a process for easily or efficiently producing a high-purity acid addition salt of a 4-amino-2-halobenzonitrile compound with an organic sulfonic acid.

### SOLUTION TO PROBLEM

The inventors of the present invention made intensive studies to achieve the above objects and finally found that, in a method of separating an acid addition salt of a 4-amino-2-halobenzonitrile compound with an acid from an acid addition salt of an isomeric mixture of an aminohalobenzonitrile compound with an acid using a difference in solubility between the isomers in a solvent, use of an organic sulfonic acid as the acid allows industrially advantageous production of an organic sulfonic acid salt of a 4-amino-2-halobenzonitrile compound, the salt having a significantly different solubility in a solvent compared with other acid addition salts. Moreover, the inventors of the present invention found that such a process can easily or efficiently produce an organic sulfonic acid salt of a 4-amino-2-halobenzonitrile compound and can easily or efficiently produce a high-purity organic sulfonic acid salt of a 4-amino-2-halobenzonitrile compound. The present invention was accomplished based on the above findings.

That is, the present invention may include the following aspects and the like.

Aspect [1]: A process for producing an acid addition salt of a 4-amino-2-halobenzonitrile compound with an organic sulfonic acid, the process comprising a separation step of separating
an acid addition salt of a 4-amino-2-halobenzonitrile compound represented by the following formula (2) with an organic sulfonic acid
from an acid addition salt of an isomeric mixture of an aminohalobenzonitrile compound represented by the following formula (1) with an organic sulfonic acid
using a difference in solubility between acid addition salts of isomers in a solvent: 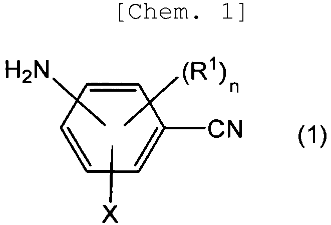
wherein
X represents a halogen atom,
R¹ represents a reactively inactive substituent, and
n denotes an integer of 0 to 3, 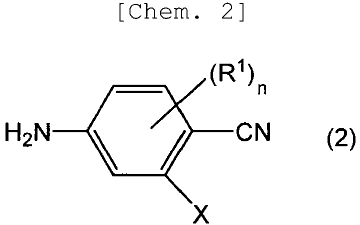
wherein
X, R¹, and n each have the same meanings as those in the formula (1).

Aspect [2]: The process of the aspect [1], wherein, in the separation step, the separation using the difference in solubility between acid addition salts of isomers in the solvent includes precipitation and/or extraction.

Aspect [3]: The process of the aspect [1] or [2], wherein, in the separation step, the solvent contains an ester and/or an alcohol.

Aspect [4]: The process of any one of the aspects [1] to [3], which comprises, as a preceding step of the separation step, a salt formation step of allowing the isomeric mixture of the aminohalobenzonitrile compound represented by the formula (1) at least containing the 4-amino-2-halobenzonitrile compound represented by the formula (2) to react with the organic sulfonic acid to form an acid addition salt.

Aspect [5]: The process of the aspect [4],
which comprises, as a preceding step of the salt formation step,
an amination step of subjecting a dihalobenzonitrile compound represented by the following formula (4) at least containing a 2,4-dihalobenzonitrile compound represented by the following formula (3) to an amination reaction: 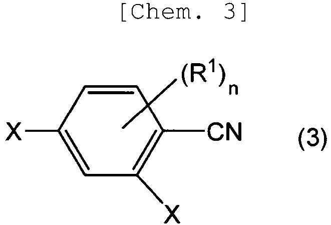
wherein
each X independently represents a halogen atom, and
R¹ and n each have the same meanings as those in the formula (1), 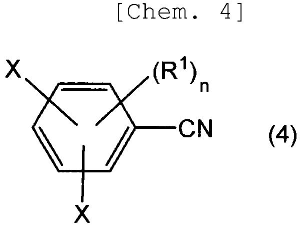
wherein
X, R¹, and n each have the same meanings as those in the formula (3).

Aspect [6]: The process of the aspect [4] or [5], wherein the reaction in the salt formation step is carried out in the presence of a solvent, the solvent used in the salt formation step is the same as the solvent used in the separation step.

Aspect [7]: The process of any one of the aspects [1] to [6], wherein the organic sulfonic acid contains an aromatic sulfonic acid.

Aspect [8]: The process of the aspect [7],
wherein the aromatic sulfonic acid contains an arene ring-containing sulfonic acid represented by the following formula (5): 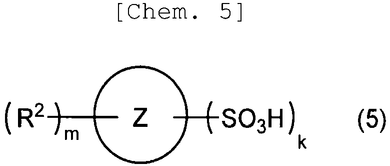
wherein
a ring Z represents an arene ring,
R² represents a reactively inactive substituent,
m denotes an integer of not less than 0, and
k denotes an integer of not less than 1.

Aspect [9]: The process of the aspect [8], wherein, in the formula (5), the ring Z represents a C₆₋₁₄arene ring, R² represents a straight- or branched-chain C₁₋₃alkyl group, m denotes 0 or 1, and k denotes 1 or 2.

Aspect [10]: The process of any one of the aspects [1] to [9], wherein a ratio of the sulfonic acid group of the organic sulfonic acid is 0.8 to 1.2 mol relative to 1 mol of a total amount of the isomeric mixture of the aminohalobenzonitrile compound represented by the formula (1).

Aspect [11]: A process for producing a 4-amino-2-halobenzamide compound represented by the following formula (6) or acid addition salt thereof: 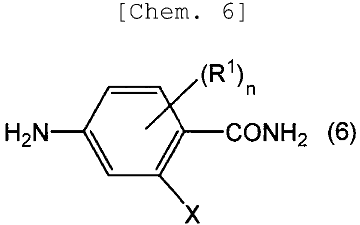 wherein
X, R¹, and n each have the same meanings as those in the formula (1) recited in the aspect [1],
the process comprising an amidation step of subjecting an acid addition salt produced by a process of any one of the aspects [1] to [10] to a hydrolysis reaction.

Aspect [12]: An acid addition salt of a 4-amino-2-halobenzonitrile compound represented by the following formula (2) with an organic sulfonic acid: 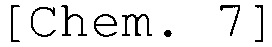 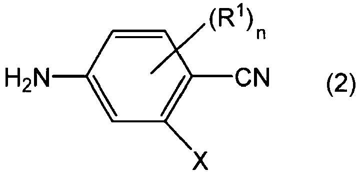 wherein
X, R¹, and n have the same meanings as those in the formula (1) recited in the aspect [1].

### ADVANTAGEOUS EFFECTS OF INVENTION

The process of the present invention allows the acid addition salt of the 4-amino-2-halobenzonitrile compound with the organic sulfonic acid to be produced industrially advantageously from the acid addition salt of the mixture of the 4-amino-2-halobenzonitrile compound and isomer(s) thereof with the organic sulfonic acid (the organic sulfonic acid salt of the mixture of the 4-amino-2-halobenzonitrile compound and isomer(s) thereof) through the separation step using a difference in solubility between acid addition salts of isomers in a solvent. Moreover, selection of a specified solvent species as the solvent or other means allows an organic sulfonic acid salt of an object compound to be produced easily or efficiently (further, if necessary, with a high purity).

### DESCRIPTION OF EMBODIMENTS

According to the present invention, through a separation step of separating an acid addition salt of a 4-amino-2-halobenzonitrile compound represented by the after-mentioned formula (2) (hereinafter, the compound may also be referred to as a "4-amino-2-halobenzonitrile compound (2)") with an organic sulfonic acid from an acid addition salt (1a) of an isomeric mixture of an aminohalobenzonitrile compound represented by the following formula (1) (hereinafter, the compound may also be referred to as an "aminohalobenzonitrile compound (1)") with an organic sulfonic acid, an acid addition salt (2a) of the 4-amino-2-halobenzonitrile compound (2) with the organic sulfonic acid is industrially advantageously produced. As a preferred aspect, the present invention may include an amination step of subjecting a dihalobenzonitrile compound represented by the following formula (4) (hereinafter, the compound may also be referred to as a "dihalobenzonitrile compound (4)") which may contain an isomeric mixture to an amination reaction to obtain an isomeric mixture of the aminohalobenzonitrile compound (1), and a salt formation step of allowing the resulting isomeric mixture of the aminohalobenzonitrile compound (1) to react with an organic sulfonic acid. The present invention includes at least the separation step.

To aid the understanding of the invention, these steps are shown in the following scheme when the organic sulfonic acid is an organic monosulfonic acid A: 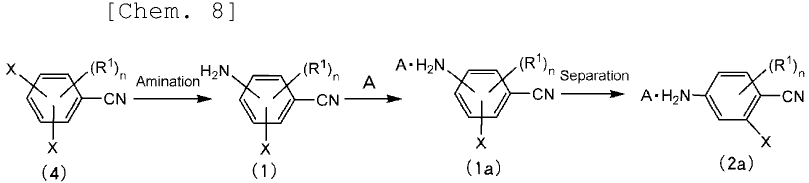 wherein
each X independently represents a halogen atom,
R¹ represents a reactively inactive substituent,
n denotes an integer of 0 to 3, and
A represents an organic monosulfonic acid.

### [Amination step]

In an amination step, the aminohalobenzonitrile compound (1) at least containing the 4-amino-2-halobenzonitrile compound (2) is produced by subjecting the dihalobenzonitrile compound (4) at least containing a 2,4-dihalobenzonitrile compound represented by the following formula (3) (hereinafter, the compound may be referred to as a "2,4-dihalobenzonitrile compound (3)") to an amination reaction to convert one halogen atom X to an amino group. 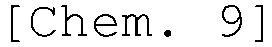 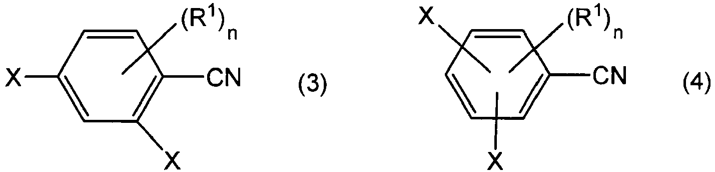 wherein
each X independently represents a halogen atom,
R¹ represents a reactively inactive substituent, and
n denotes an integer of 0 to 3, 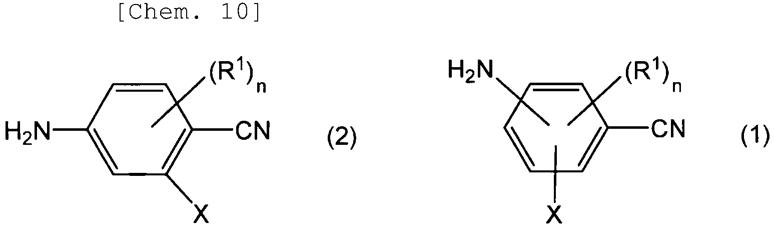
wherein
X represents a halogen atom, and
R¹ and n have the same meanings as those in the formula (3).

In the formulae (1), (2), (3) and (4), examples of the halogen atom represented by X may include a fluorine atom, a chlorine atom, a bromine atom, and an iodine atom.

Among these halogen atoms, a fluorine atom, a chlorine atom, or a bromine atom is preferred, a fluorine atom or a chlorine atom is further preferred, and a fluorine atom is more preferred.

In the formulae (3) and (4), the species of two atoms X may be different from each other, and are preferably the same.

In the formulae (1), (2), (3), and (4), the substituent R¹ is not particularly limited and is any reactively inactive group. Examples of the substituent may include a hydroxy group, a straight- or branched-chain alkyl group (such as a C₁₋₆alkyl group such as methyl group, ethyl group, propyl group, isopropyl group, butyl group, isobutyl group, sec-butyl group, and tert-butyl group), a straight-or branched-chain alkoxy group (such as a C₁₋₆alkoxy group such as methoxy group, ethoxy group, and propoxy group), and a cycloalkyl group (such as a C₃₋₁₂cycloalkyl group such as cyclopropyl group, cyclobutyl group, and cyclohexyl group).

Among these substituents R¹, preferred is a straight- or branched-chain alkyl group, a straight- or branched-chain alkoxy group, or a cycloalkyl group, further preferred is a straight- or branched-chain alkyl group (such as a straight- or branched-chain C₁₋₆alkyl group), and more preferred is a straight- or branched-chain C₁₋₃alkyl group such as methyl group and ethyl group.

In the formulae (1), (2), (3), and (4), the number (substitution number) n of substituents R¹ is an integer of 0 to 3, preferably an integer of 0 to 2, further preferably 0 or 1, and more preferably 0. In a case where the number n of substituents R¹ is an integer of not less than 2, the species of the two or more substituents R¹ may be different from each other or may be the same.

In the formula (3), the substitution position(s) of the substituent R¹ is any of 3-position, 5-position, and/or 6-position with respect to cyano group on 1-position of the 2,4-dihalobenzonitrile compound (3).

Representative examples of the 2,4-dihalobenzonitrile compound (3) may include a 2,4-dihalobenzonitrile such as 2,4-difluorobenzonitrile, 2,4-dichlorobenzonitrile, and 2,4-dibromobenzonitrile.

In the formula (4), the substitution positions of two halogen atoms X are not particularly limited and are any positions that contain a combination of 2-position and 4-position corresponding to the 2,4-dihalobenzonitrile compound (3). The substitution positions may contain a combination of 2-position and 4-position and a combination of any two of 2-position, 3-position, 4-position, 5-position, and 6-position (excluding the combination of 2-position and 4-position) with respect to cyano group on 1-position of the dihalobenzonitrile compound (4). The proportion of the 2,4-dihalobenzonitrile compound (3) in the dihalobenzonitrile compound (4) is, for example, not less than 50%, preferably not less than 60% (such as not less than 70%), further preferably not less than 80% (such as not less than 90%), most preferably 100% (that is, the dihalobenzonitrile compound (4) contains only the 2,4-dihalobenzonitrile compound (3)).

In the formula (4), the substitution position(s) of the substituent R¹ is any position(s) that is different from the substitution positions of the halogen atoms X.

Representative examples of the dihalobenzonitrile compound (4) may include a dihalobenzonitrile such as a difluorobenzonitrile, a dichlorobenzonitrile, and a dibromobenzonitrile.

As examples of an agent for amination (an amination agent), there may be mentioned ammonia, ammonia water, and a metal amide salt (such as an alkali metal amide such as lithium amide, potassium amide, and sodium amide). Among these amination agents, preferred is ammonia, ammonia water, or an alkali metal amide, further preferred is ammonia or ammonia water, and more preferred is ammonia water.

These amination agents may be used alone or in combination of two or more.

The ratio of the amination agent relative to 1 mol of the dihalobenzonitrile compound (4) is, for example, 1 to 10 mol, preferably 2 to 8 mol, further preferably 3 to 7 mol, and more preferably 4 to 6 mol. An excessively low ratio of the amination agent may cause a low yield.

In the amination step, the reaction of the dihalobenzonitrile compound (4) with the amination agent may be carried out in the presence or absence of a solvent.

As the solvent, a polar solvent in which the amination agent can be dissolved is preferred. For example, an aprotic polar organic solvent may be used.

Examples of the aprotic polar organic solvent may include a sulfoxide (such as a diC₁₋₆alkyl sulfoxide such as dimethyl sulfoxide), a sulfone (such as a cyclic sulfone such as sulfolane), an amide [for example, a chain amide (such as an N,N-diC₁₋₆alkyl-C₁₋₆acylamide such as N,N-dimethylformamide and N,N-dimethylacetamide), and a cyclic amide (such as N-methyl-2-pyrrolidone)], an ether (such as a chain ether such as diethyl ether, and a cyclic ether such as tetrahydrofuran), a ketone (such as acetone and methyl ethyl ketone), an ester (such as ethyl acetate), and a nitrile (such as acetonitrile).

These aprotic polar organic solvents may be used alone or in combination of two or more. Among them, preferred is a sulfoxide, further preferred is a diC₁₋₆alkyl sulfoxide (such as a diC₁₋₄alkyl sulfoxide), and more preferred is dimethyl sulfoxide.

The aprotic polar organic solvent has any ratio that allows the reaction system to be stirred. The ratio of the aprotic polar organic solvent relative to 100 parts by mass of the dihalobenzonitrile compound (4) is, for example, 50 to 500 parts by mass, preferably 100 to 400 parts by mass, further preferably 150 to 300 parts by mass, and more preferably 200 to 250 parts by mass. An excessively low ratio of the solvent may make the stirring of the reaction system difficult. An excessively high ratio of the solvent may make the progress of the reaction slow.

Moreover, as the solvent, water may be contained in the form of an aqueous solution of the amination agent.

The ratio of water as the solvent relative to 100 parts by mass of the dihalobenzonitrile compound (4) is, for example, 10 to 300 parts by mass, preferably 50 to 250 parts by mass, further preferably 100 to 200 parts by mass, and more preferably 140 to 180 parts by mass.

The ratio of water as the solvent relative to 100 parts by mass of the organic solvent is, for example, 10 to 200 parts by mass, preferably 30 to 150 parts by mass, further preferably 40 to 100 parts by mass, and more preferably 60 to 80 parts by mass.

The reaction temperature is, for example, 50 to 180°C, preferably 70 to 150°C, further preferably 90 to 130°C, and more preferably 95 to 120°C. The reaction time is not particularly limited to a specific time and is, for example, 2 to 40 hours, preferably 8 to 30 hours, further preferably 12 to 25 hours, and more preferably 15 to 21 hours.

The reaction may be carried out in air or in an inert gas (such as nitrogen; and a rare or noble gas such as argon and helium). The reaction may be carried out under a normal or ordinary pressure or under an applied pressure.

In the formula (2), the substitution position(s) of the substituent R¹ is the same as the substitution position(s) in the formula (3).

Representative examples of the 4-amino-2-halobenzonitrile compound (2) may include a 4-amino-2-halobenzonitrile such as 4-amino-2-fluorobenzonitrile, 4-amino-2-chlorobenzonitrile, and 4-amino-2-bromobenzonitrile.

In the formula (1), the substitution position of the halogen atom X is not particularly limited to a specific position and may be any one of 2-position, 3-position, 4-position, 5-position, and 6-position with respect to cyano group on 1-position of the aminohalobenzonitrile compound (1). Among them, preferred is 2-position or 4-position.

In the formula (1), the substitution position of the amino group is not particularly limited to a specific position and may be any one of 2-position, 3-position, 4-position, 5-position, and 6-position with respect to cyano group on 1-position of the aminohalobenzonitrile compound (1). Among them, preferred is 2-position or 4-position.

Specifically, the aminohalobenzonitrile compound (1) preferably contains at least the 4-amino-2-halobenzonitrile compound (2) and a 2-amino-4-halobenzonitrile compound, further preferably contains the 4-amino-2-halobenzonitrile compound (2) and the 2-amino-4-halobenzonitrile compound as main components, and particularly preferably contains only the 4-amino-2-halobenzonitrile compound (2) and the 2-amino-4-halobenzonitrile compound.

The organic sulfonic acid salt of the 4-amino-2-halobenzonitrile compound (2) seems to have a significantly lowered solubility in a solvent compared with other isomers such as an organic sulfonic acid salt of the 2-amino-4-halobenzonitrile compound. In the after-mentioned separation step, since the acid addition salt of the 4-amino-2-halobenzonitrile compound (2) is obtained using the above-mentioned difference in solubility, the aminohalobenzonitrile compound (1) containing the 4-amino-2-halobenzonitrile compound (2) and the 2-amino-4-halobenzonitrile compound as main components allows an improved separation efficiency of the acid addition salt.

In the formula (1), the substitution position(s) of the substituent R¹ is any position(s) different from the substitution positions of the halogen atom X and the amino group.

Representative examples of the aminohalobenzonitrile compound (1) may include a 4-amino-2-halobenzonitrile such as 4-amino-2-fluorobenzonitrile, 4-amino-2-chlorobenzonitrile, and 4-amino-2-bromobenzonitrile, and a 2-amino-4-halobenzonitrile such as 2-amino-4-fluorobenzonitrile, 2-amino-4-chlorobenzonitrile, and 2-amino-4-bromobenzonitrile.

### [Salt formation step]

In the salt formation step, the isomeric mixture of the aminohalobenzonitrile compound (1) containing at least the 4-amino-2-halobenzonitrile compound (2) is allowed to react with an organic sulfonic acid to form an acid addition salt of the isomeric mixture (an organic sulfonic acid salt of the mixture).

In the present invention, a means such as selection of an organic sulfonic acid as an acid can improve the efficiency of separating the objective acid addition salt of the 4-amino-2-halobenzonitrile compound (2) from the acid addition salt of the isomeric mixture in the after-mentioned separation step.

The organic sulfonic acid is not particularly limited, and is any organic sulfonic acid that has one or more sulfonic acid groups in a molecule thereof, can form a salt with the 4-amino-2-halobenzonitrile compound (1), and allows separation of the isomer(s). Examples of the organic sulfonic acid may include a sulfonic acid having no aromatic ring in a molecule thereof and having an aliphatic hydrocarbon group (an aliphatic sulfonic acid), and a sulfonic acid having at least one aromatic ring in a molecule thereof (an aromatic sulfonic acid).

These organic sulfonic acids may be used alone or in combination of two or more.

As examples of the aliphatic sulfonic acid, there may be mentioned an alkanesulfonic acid (such as a C₁₋₁₂alkanesulfonic acid such as methanesulfonic acid, ethanesulfonic acid, and dodecanesulfonic acid), an alkenylsulfonic acid (such as a C₂₋₁₂alkenylsulfonic acid such as vinylsulfonic acid and butenylsulfonic acid), and a halogenated sulfonic acid (such as a trihaloC₁₋₆alkanesulfonic acid such as trichloromethanesulfonic acid, tribromomethanesulfonic acid, and trifluoromethanesulfonic acid).

These aliphatic sulfonic acids may be used alone or in combination of two or more.

Examples of the aromatic ring of the aromatic sulfonic acid may include an aromatic heterocycle that contains any of a nitrogen atom, an oxygen atom, and a sulfur atom as a constituent atom of the ring, and an arene ring. These aromatic sulfonic acids may be used alone or in combination of two or more.

As examples of the aromatic heterocycle, there may be mentioned a heterocycle containing at least one heteroatom, such as a nitrogen-containing heterocycle (such as a pyridine ring, a quinoline ring, a carbazole ring, an imidazole ring, and phthalimide ring) and a nitrogen- and oxygen-containing heterocycle (such as an oxazole ring and an isoxazole ring).

The arene ring may be either a monocyclic arene ring (such as a benzene ring) or a polycyclic arene ring (such as a fused or condensed polycyclic arene ring and a ring-assembly or ring-aggregated arene ring).

Examples of the fused polycyclic arene ring may include a fused bicyclic arene ring (such as a fused bicyclic C₁₀₋₂₀arene ring such as a naphthalene ring and an indene ring) and a fused tricyclic arene ring (such as a fused tricyclic C₁₄₋₂₀arene ring such as an anthracene ring and a phenanthrene ring).

As examples of the ring-assembly arene ring, there may be mentioned a biarene ring (such as a C₁₂₋₂₀biarene ring such as a biphenyl ring, a phenylnaphthalene ring, and a binaphthyl ring).

Among these aromatic rings, preferred is a monocyclic arene ring (such as a benzene ring), a fused polycyclic arene ring (such as a fused bicyclic C₁₀₋₂₀arene ring such as a naphthalene ring and an indene ring, and a fused tricyclic C₁₄₋₂₀arene ring such as an anthracene ring and a phenanthrene ring), further preferred is a monocyclic arene ring or a fused bicyclic arene ring, more preferred is a benzene ring or a naphthalene ring, and most preferred is a benzene ring.

In the aromatic sulfonic acid, the aromatic ring and the sulfonic acid group may be bonded directly or may be bonded through a linking group (such as an alkylene group), and are preferably bonded directly.

Examples of the aromatic sulfonic acid in which the aromatic ring is an arene ring may include a compound represented by the following formula (5): 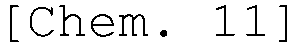 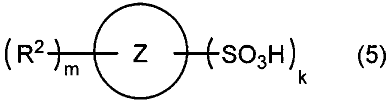 wherein
a ring Z represents an arene ring,
R² represents a reactively inactive substituent,
m denotes an integer of not less than 0, and
k denotes an integer of not less than 1.

Examples of the ring Z may include the arene ring, such as the monocyclic arene ring and the polycyclic arene ring, exemplified as the aromatic ring in the aromatic sulfonic acid.

Among these arene rings, preferred is a C₆₋₂₀arene ring, further preferred is a C₆₋₁₄arene ring, more preferred is a C₆₋₁₂arene ring, particularly preferred is a C₆₋₁₀arene ring such as a benzene ring and a naphthalene ring, and most preferred is a benzene ring.

The substituent R² is not particularly limited and is any reactively inactive group. Examples of the substituent R² may include a hydroxy group, a halogen atom (such as a fluorine atom, a chlorine atom, a bromine atom, and an iodine atom), a nitro group, a straight- or branched-chain alkyl group (such as a C₁₋₁₂alkyl group such as methyl group, ethyl group, and propyl group), a straight- or branched-chain alkoxy group (such as a C₁₋₁₂alkoxy group such as methoxy group, ethoxy group, and propoxy group), and a cycloalkyl group (such as a C₃₋₁₄cycloalkyl group such as cyclopropyl group, cyclobutyl group, and cyclohexyl group). Among them, preferred is an alkyl group (such as a straight- or branched-chain C₁₋₆alkyl group such as methyl group, ethyl group, propyl group, isopropyl group, butyl group, isobutyl group, pentyl group, and hexyl group), an alkoxy group (such as a straight- or branched-chain C₁₋₆alkoxy group such as methoxy group, ethoxy group, propoxy group, and isopropoxy group), or a cycloalkyl group (such as a C₃₋₁₂cycloalkyl group such as cyclobutyl group and cyclohexyl group), further preferred is a straight- or branched-chain C₁₋₄alkyl group such as methyl group, ethyl group, propyl group, and butyl group, and more preferred is a straight- or branched-chain C₁₋₃alkyl group such as methyl group and ethyl group.

The number (substitution number) m of substituents R² is an integer of not less than 0, preferably an integer of 0 to 3, further preferably an integer of 0 to 2, more preferably 0 or 1, and most preferably 1. In a case where the number m of substituents R² is an integer of not less than 2, the species of the two or more substituents R² may be different from each other or may be the same. Moreover, the substitution position(s) of the substituent R² is not particularly limited to specific position(s). In a case where the ring Z represents a benzene ring and m denotes 1, the substituent R² preferably presents at para position with respect to a sulfonic acid group.

The number (substation number) k of sulfonic acid groups is an integer of not less than 1, preferably an integer of 1 to 3, and further preferably 1 or 2. In terms of the purity of the resulting product, the number k is more preferably 1.

Representative examples of the compound represented by the formula (5) may include a benzenemono- or di-sulfonic acid which may have one or more (such as 2 or 3) alkyl group(s), such as benzenesulfonic acid and p-toluenesulfonic acid; and a naphthalenemono- or di-sulfonic acid which may have one or more (such as 2 or 3) alkyl group(s), such as 1-naphthalenesulfonic acid, 2-naphthalenesulfonic acid, 1,5-naphthalenedisulfonic acid, and 2,6-naphthalenedisulfonic acid.

These aromatic sulfonic acids represented by the formula (5) may be used alone or in combination of two or more.

Among these organic sulfonic acids, from the point of view such as a separation efficiency of the acid addition salt with the 4-amino-2-halobenzonitrile compound (2) and a productivity, preferred is an aromatic sulfonic acid; further preferred is an aromatic sulfonic acid represented by the formula (5); more preferred is an aromatic sulfonic acid represented by the formula (5) in which the ring Z represents a C₆₋₁₄arene ring, R² represents an alkyl group, m denotes an integer of 0 to 2, and k denotes an integer of 1 to 3; more preferred is a benzenemono- or di-sulfonic acid which may have a C₁₋₆alkyl group, such as benzenesulfonic acid and p-toluenesulfonic acid, or a naphthalenemono- or di-sulfonic acid which may have a C₁₋₆alkyl group, such as 1-naphthalenesulfonic acid, 2-naphthalenesulfonic acid, and 1,5-naphthalenedisulfonic acid; particularly preferred is a benzenemonosulfonic acid which may have a C₁₋₃alkyl group, such as benzenesulfonic acid and p-toluenesulfonic acid, or a naphthalenemonosulfonic acid which may have a C₁₋₃alkyl group, such as 1-naphthalenesulfonic acid and 2-naphthalenesulfonic acid; and most preferred is a benzenemonosulfonic acid which may have a C₁₋₃alkyl group, such as p-toluenesulfonic acid.

Regarding the ratio of the organic sulfonic acid (in particular, the aromatic sulfonic acid represented by the formula (5)), the sulfonic acid group of the organic sulfonic acid relative to 1 mol of the total amount of the isomeric mixture of the aminohalobenzonitrile compound (1) is, for example, 0.1 to 3 mol, preferably 0.4 to 2 mol, further preferably 0.6 to 1.5 mol, and more preferably 0.8 to 1.2 mol. An excessively low ratio of the sulfonic acid group may reduce the yield of the objective product. An excessively high ratio of the sulfonic acid group may reduce the purity of the objective product.

The reaction that forms the acid addition salt may be carried out either in the presence or absence of a solvent, and may preferably be carried out in the presence of the solvent.

The solvent may be an organic solvent and may be an organic solvent containing water. For example, water may be contained in the form of a hydrate of an organic sulfonic acid.

As examples of the organic solvent, there may be mentioned an aliphatic hydrocarbon, an alicyclic hydrocarbon, an aromatic hydrocarbon, a halogenated hydrocarbon, an ether, a ketone, a sulfoxide, a sulfone, an amide, a nitrile, an ester, and an alcohol. These solvents may be either a polar solvent or a non-polar solvent.

Examples of the aliphatic hydrocarbon may include a straight- or branched-chain C₅₋₁₅alkane such as pentane, hexane, heptane, octane, nonane, and decane.

As examples of the alicyclic hydrocarbon, there may be mentioned a C₅₋₁₅cycloalkane such as cyclopentane, cyclohexane, and cyclooctane.

Examples of the aromatic hydrocarbon may include benzene, and a mono- or di-C₁₋₆alkylbenzene such as toluene and xylene.

As examples of the halogenated hydrocarbon, there may be mentioned a chlorohydrocarbon such as methylene chloride, chloroform, carbon tetrachloride, dichloroethane, chlorobenzene, and dichlorobenzene.

Examples of the ether may include a chain ether (such as diethyl ether), and a cyclic ether (such as tetrahydrofuran).

As examples of the ketone, there may be mentioned a chain ketone (such as a straight- or branched-chain diC₁₋₁₂alkyl-ketone such as acetone, methyl ethyl ketone, methyl isobutyl ketone, and diisobutyl ketone), and a cyclic ketone (such as cyclohexanone).

Examples of the sulfoxide may include a dialkyl sulfoxide (such as a diC₁₋₆alkyl-sulfoxide such as dimethyl sulfoxide).

As examples of the sulfone, there may be mentioned a cyclic sulfone such as sulfolane.

Examples of the amide may include a chain amide such as an N,N-dialkylacylamide (such as an N,N-diC₁₋₆alkyl-C₁₋₆acylamide such as N,N-dimethylformamide, N,N-diethylformamide, N,N-dimethylacetamide, and N,N-diethylacetamide), and a cyclic amide such as N-methyl-2-pyrrolidone.

As examples of the nitrile, there may be mentioned a C₁₋₆alkyl-nitrile such as acetonitrile and propionitrile, and a C₆₋₁₂arene-nitrile such as benzonitrile.

Examples of the ester may include a chain ester [for example, a carboxylate ester (such as an alkanoic acid ester) such as a formate ester, an acetate ester, a propionate ester, and a butyrate ester; a hydroxycarboxylate ester (such as a hydroxyalkanoic acid ester) such as a lactate ester; and an ether ester (such as a C₂₋₁₀alkylene glycol monoC₁₋₁₀alkyl ether C₁₋₁₀acylate such as ethylene glycol monomethyl ether acetate and propylene glycol monomethyl ether acetate)], and a cyclic ester (such as a three-membered ring lactone, a four-membered ring lactone, a five-membered ring lactone, and a six-membered ring lactone).

As examples of the formate ester, there may be mentioned an alkyl formate (such as a C₁₋₆alkyl formate such as methyl formate, ethyl formate, propyl formate, and butyl formate).

Examples of the acetate ester may include an alkyl acetate (such as a C₁₋₆alkyl acetate such as methyl acetate, ethyl acetate, propyl acetate, and butyl acetate).

As examples of the propionate ester, there may be mentioned an alkyl propionate (such as a C₁₋₆alkyl propionate such as methyl propionate, ethyl propionate, propyl propionate, and butyl propionate).

Examples of the butyrate ester may include an alkyl butyrate (such as a C₁₋₆alkyl butyrate such as ethyl butyrate, isopropyl butyrate, and butyl butyrate).

As examples of the lactate ester, there may be mentioned an alkyl lactate (such as a C₁₋₆alkyl lactate such as methyl lactate, ethyl lactate, and butyl lactate).

Examples of the cyclic ester may include a C₂₋₁₀cyclic ester such as β-propiolactone, γ-butyrolactone, δ-valerolactone, and ε-caprolactone.

As examples of the alcohol, there may be mentioned a straight- or branched-chain alcohol (such as a straight- or branched-chain C₁₋₁₂alkanol such as methanol, ethanol, n-propanol, isopropanol (isopropyl alcohol), n-butanol, isobutanol, sec-butanol, tert-butanol, n-pentanol, isopentanol, 2-methylbutanol, sec-pentanol, and tert-pentanol), and an ether alcohol (such as a C₂₋₁₀alkylene glycol mono- or di-C₁₋₁₀alkyl ether such as 2-methoxyethanol, 2-methoxypropanol, 2-ethoxyethanol, and propylene glycol monomethyl ether).

These organic solvents may be used alone or in combination of two or more.

Among these organic solvents, in terms of the ease of use as a solvent for separating the objective compound in the after-mentioned separation step, preferred is an ether, a ketone, an ester, or an alcohol, further preferred is an ester [for example, an alkyl carboxylate (such as an alkyl alkanoate) such as an alkyl acetate] or an alcohol (such as a straight- or branched-chain alcohol), more preferred is a C₁₋₆alkyl acetate such as methyl acetate, ethyl acetate, and propyl acetate, or a straight- or branched-chain C₁₋₆alkanol such as methanol, ethanol, n-propyl alcohol, and isopropyl alcohol, particularly preferred is a C₁₋₃alkyl acetate such as methyl acetate and ethyl acetate, or a C₁₋₃alkanol such as methanol, ethanol, and isopropyl alcohol, and most preferred is isopropyl alcohol.

The solvent has any ratio that allows the reaction system to be stirred. The ratio of the solvent relative to 100 parts by mass of the total amount of the isomeric mixture of the aminohalobenzonitrile compound (1) is, for example, 50 to 500 parts by mass, preferably 100 to 400 parts by mass, further preferably 150 to 350 parts by mass, and more preferably 200 to 300 parts by mass. An excessively low ratio of the solvent may make the stirring of the reaction system difficult. An excessively high ratio of the solvent may make the progress of the reaction slow.

The reaction temperature is, for example, 0 to 50°C, preferably 10 to 40°C, further preferably 15 to 35°C, and more preferably 20 to 30°C. The reaction time is not particularly limited to a specific time and is, for example, 0.5 to 5 hours, and preferably 1 to 2 hours.

The reaction may be carried out in air or in an inert gas (such as nitrogen; and a rare or noble gas such as argon and helium). The reaction may be carried out under a normal or ordinary pressure or under an applied pressure.

### [Separation step]

In the separation step, from the acid addition salt of the isomeric mixture of the aminohalobenzonitrile compound (1) with the organic sulfonic acid (a mixture containing an organic sulfonic acid salt of the objective compound and organic sulfonic acid salts of other isomers), the acid addition salt of the 4-amino-2-halobenzonitrile compound (2) with the organic sulfonic acid (the organic sulfonic acid salt of the objective compound) is separated using a difference in solubility between the acid addition salts in the mixture in a solvent.

Examples of the separation method using the difference in solubility in the solvent may include precipitation (or crystallization), solvent extraction (or elution), and a combination thereof.

These methods may be used alone or in combination with one or more conventional separation means selected from washing, concentration, solid-liquid separation (such as filtration, centrifugation, and decantation), column chromatography, and other means.

The organic sulfonic acid salt of the objective compound may be obtained as a liquid phase or may be obtained as a solid phase. In terms of the productivity, it is preferred that the organic sulfonic acid salt be obtained as a solid phase.

The precipitation (or crystallization) may be carried out by a conventional means. In the precipitation (or crystallization), the organic sulfonic acid salt of the objective compound is separated from the mixture by precipitating the organic sulfonic acid salt of the objective compound as a solid product in a precipitation solvent (a crystallization solvent) while dissolving the organic sulfonic acid salts of other isomers in the precipitation solvent (the crystallization solvent).

Examples of the precipitation (or crystallization) method may include cooling crystallization, anti-solvent crystallization, concentration crystallization, reprecipitation, and a combination thereof. Among them, cooling crystallization is preferred.

The solvent extraction (or elution) may be carried out by a conventional means. In the solvent extraction (or elution), the isomeric mixture containing the organic sulfonic acid salt of the objective compound is concentrated or dried for solidification, and then the organic sulfonic acid salt of the objective compound is separated from the mixture by extracting the organic sulfonic acid salts of other isomers into a liquid phase using an extraction solvent (an elution solvent).

The precipitation solvent (the crystallization solvent) and the extraction solvent (the elution solvent) contain at least an organic solvent, and may contain water. For example, water may be added in the form of the hydrate of the organic sulfonic acid in the salt formation step.

Examples of the organic solvent may include an organic solvent exemplified in the salt formation step.

These organic solvents may be used alone or in combination of two or more.

Among these organic solvents, preferred is an ether, a ketone, an ester, or an alcohol, and in terms of the productivity, further preferred is an ester [for example, an alkyl carboxylate (such as an alkyl alkanoate) such as an alkyl acetate] or an alcohol (such as a straight- or branched-chain alcohol), more preferred is a C₁₋₆alkyl acetate such as methyl acetate, ethyl acetate, and propyl acetate, or a straight- or branched-chain C₁₋₆alcohol (such as a C₁₋₆alkanol) such as methanol, ethanol, n-propyl alcohol, and isopropyl alcohol, particularly preferred is a C₁₋₃alkyl acetate such as methyl acetate and ethyl acetate, or a C₁₋₃alcohol (such as a C₁₋₃alkanol) such as methanol, ethanol, and isopropyl alcohol, and most preferred is isopropyl alcohol.

Among these organic solvents, from the viewpoint of the purity of the product, preferred is an alcohol, more preferred is methanol, ethanol, n-propyl alcohol, or isopropyl alcohol, and most preferred is isopropyl alcohol.

In the precipitation (or crystallization), the precipitation solvent (the crystallization solvent) may be different from the reaction solvent used in the salt formation step, and is preferably the same as the reaction solvent. In a case where the precipitation solvent is the same as the reaction solvent, the reaction solvent can be used as it is as the precipitation solvent (the crystallization solvent), resulting in an improved productivity.

The concentration of the acid addition salt in the solution to be subjected to the precipitation (or crystallization) method (in particular, cooling crystallization) is, for example, 5 to 80% by mass, preferably 10 to 65% by mass, further preferably 30 to 60% by mass, and more preferably 40 to 55% by mass. An excessively low concentration of the acid addition salt may cause a low yield. An excessively high concentration of the acid addition salt may make it difficult to maintain a stirring uniformity and a temperature gradient in a crystallization tank.

The cooling temperature is not particularly limited and is any temperature that allows the organic sulfonic acid salt of the objective compound to precipitate. The cooling temperature may for example be -20°C to 25°C, and is preferably -10°C to 20°C, further preferably -5°C to 10°C, and more preferably 0 to 5°C.

For the cooling crystallization, the cooling rate is not particularly limited to a specific rate and is, for example, 0.01 to 8°C/min., preferably 0.05 to 5°C/min., further preferably 0.1 to 2°C/min., and more preferably 0.5 to 1°C/min. An excessively high cooling rate may reduce the purity of the objective product. An excessively low cooling rate may reduce the productivity.

Further solid-liquid separation (such as filtration, centrifugation, and decantation) to be carried out after the precipitation (or crystallization) can obtain the separated organic sulfonic acid salt of the objective compound.

The precipitation (or crystallization) operation may be carried out once or may be repeated multiple times. For repeating multiple times, the precipitation solvent (the crystallization solvent) after the solid-liquid separation may be concentrated for further precipitation (such as cooling crystallization), thereby collecting the organic sulfonic acid salt of the objective compound.

The resulting organic sulfonic acid salt of the objective compound may be purified by one or more conventional methods selected from washing with the precipitation solvent or the like, distillation, sublimation, column chromatography, and the like.

In the extraction (or elution), the reaction solvent used in the salt formation step is concentrated or dried for solidification, and the extraction solvent may be the same as or different from the reaction solvent.

The ratio of the extraction solvent relative to 100 parts by mass of the isomeric mixture containing the organic sulfonic acid salt of the objective compound is, for example, 1 to 50 parts by mass, and is preferably 5 to 40 parts by mass, further preferably 10 to 35 parts by mass, and more preferably 15 to 30 parts by mass.

The concentration of the isomeric mixture containing the organic sulfonic acid salt of the objective compound after the concentration or drying for solidification is, for example, not less than 70% by mass, and is preferably not less than 85% by mass, further preferably not less than 95% by mass, and more preferably not less than 99% by mass. An excessively low concentration of the mixture may cause a low yield.

The temperature of the solvent extraction (or elution) is not particularly limited and is any temperature that allows the organic sulfonic acid salts of other isomers to be extracted (or eluted) into a liquid phase using the extraction solvent. The temperature is, for example, 0 to 50°C, and is preferably 5 to 45°C, further preferably 10 to 40°C, and more preferably 20 to 35°C.

Further solid-liquid separation (such as filtration, centrifugation, and decantation) to be carried out after the extraction (or elution) can obtain the separated organic sulfonic acid salt of the objective compound.

The solvent extraction (or elution) operation may be carried out once or may be repeated multiple times. For repeating multiple times, the extraction solvent after the solid-liquid separation may be concentrated or dried for solidification, thereby collecting a solid phase, and then the solid phase may be subjected to further extraction (or elution). In a case where the extraction (or elution) is carried out multiple times, the species of the solvents to be used may be the same or may be different from each other.

The resulting organic sulfonic acid salt of the objective compound may be purified by one or more conventional methods selected from washing with the extraction solvent or the like, distillation, sublimation, column chromatography, and the like.

The precipitation (or crystallization) and the solvent extraction (or elution) may be used alone or may be used in combination thereof.

Among these separation methods, the precipitation (or crystallization) is preferred from the viewpoint that continuous carrying out of the salt formation step and the separation step by use of the reaction solvent as the precipitation solvent can improve the productivity.

### [Acid addition salt of 4-amino-2-halobenzonitrile compound with organic sulfonic acid]

The present invention includes an acid addition salt of a 4-amino-2-halobenzonitrile compound (2) represented by the following formula (2) with an organic sulfonic acid: 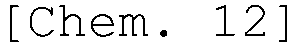 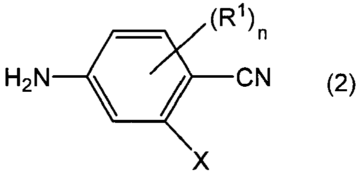 wherein
X represents a halogen atom,
R¹ represents a reactively inactive substituent, and
n denotes an integer of 0 to 3.

In the formula (2), the halogen atom X, the substituent R¹, and the integer n are the same as the halogen atom X, the substituent R¹, and the integer n shown in the above-mentioned separation step, including preferred aspects.

Representative examples of the compound represented by the formula (2) may include 4-amino-2-fluorobenzonitrile, 4-amino-2-chlorobenzonitrile, and 4-amino-2-bromobenzonitrile.

The organic sulfonic acid is the same as that shown in the salt formation step, including preferred aspects, and is preferably the aromatic sulfonic acid represented by the formula (5).

Representative examples of the acid addition salt of the 4-amino-2-halobenzonitrile compound (2) with the organic sulfonic acid may include an acid addition salt of the 4-amino-2-halobenzonitrile compound (2) with the aromatic sulfonic acid represented by the formula (5), including an acid addition salt of a 4-amino-2-halobenzonitrile with a C₆₋₁₀arenemono- or di-sulfonic acid which may have a C₁₋₆alkyl group, such as an acid addition salt of 4-amino-2-fluorobenzonitrile with at least one acid selected from benzenesulfonic acid, p-toluenesulfonic acid, 1-naphthalenesulfonic acid, 2-naphthalenesulfonic acid, 1,5-naphthalenedisulfonic acid, and 2,6-naphthalenedisulfonic acid.

Such an acid addition salt of the 4-amino-2-halobenzonitrile compound with the organic sulfonic acid is useful as a pharmaceutical intermediate, a reagent, and the like.

### [Amidation step]

In the present invention, as a post-separation step, an amidation step of producing a 4-amino-2-halobenzamide compound represented by the following formula (6) (hereinafter, the compound may simply be referred to as a "4-amino-2-halobenzamide compound (6)") or an organic sulfonic acid salt thereof may be included.

The amidation step is shown in the following scheme: 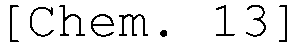 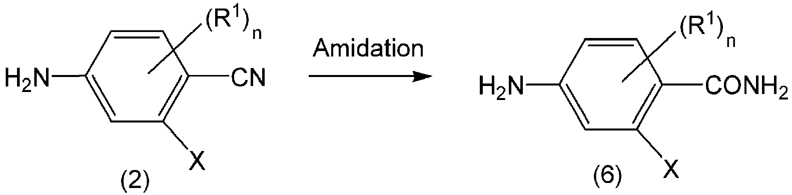 wherein
X, R¹, and n are the same as those in the formula (2), including preferred aspects.

In the amidation step, the compounds represented by the formulae (2) and (6) may be in the form of organic sulfonic acid salts. In terms of the productivity, it is preferred that the 4-amino-2-halobenzonitrile compound (2) in a salt form be subjected to the amidation reaction.

In the amidation step, the 4-amino-2-halobenzonitrile compound (2) or the organic sulfonic acid salt thereof is subjected to a hydrolysis reaction to produce the 4-amino-2-halobenzamide compound (6) represented by the formula (6) or the organic sulfonic acid salt thereof.

Representative examples of the 4-amino-2-halobenzamide compound (6) may include a 4-amino-2-halobenzamide such as 4-amino-2-fluorobenzamide, 4-amino-2-chlorobenzamide, and 4-amino-2-bromobenzamide.

The hydrolysis reaction may be carried out in the presence of a base or an acid. Among them, it is preferred that the hydrolysis reaction be carried out in the presence of a base.

Examples of the base in the hydrolysis reaction may include an alkali metal hydroxide (such as lithium hydroxide, potassium hydroxide, and sodium hydroxide), an alkaline earth metal hydroxide (such as magnesium hydroxide and calcium hydroxide), and ammonia (ammonia water). These bases may be used alone or in combination of two or more.

Among them, preferred is an alkali metal hydroxide, further preferred is sodium hydroxide or potassium hydroxide, and more preferred is sodium hydroxide.

The ratio of the base in the hydrolysis reaction relative to 1 mol of the 4-amino-2-halobenzonitrile compound (2) is, for example, 0.3 to 10 mol, preferably 0.5 to 6 mol, further preferably 0.8 to 3 mol, and more preferably 0.9 to 1.5 mol. The ratio of the base relative to 1 equivalent of the 4-amino-2-halobenzonitrile compound (2) is, for example, 1 to 8 equivalents, preferably 1.05 to 5 equivalents, further preferably 1.1 to 2 equivalents, and more preferably 1.2 to 1.3 equivalents. An excessively low ratio of the base may make the reaction rate slow. An excessively high ratio of the base may produce many impurities as by-products.

The hydrolysis reaction is preferably carried out in the presence of a peroxide under a basic condition in terms of improving the yield of the objective product by stopping the progress of the hydrolysis at the stage of amide group formation. The peroxide may be an inorganic peroxide or may be an organic peroxide.

Examples of the inorganic peroxide may include hydrogen peroxide, an alkali metal peroxide (such as sodium peroxide and lithium peroxide), an alkaline earth metal peroxide (such as magnesium peroxide and calcium peroxide), and a persulfate (such as ammonium persulfate and potassium persulfate).

As examples of the organic peroxide, there may be mentioned a peracid (such as peracetic acid, perbenzoic acid, and perchlorobenzoic acid), and a hydroperoxide (such as t-butyl hydroperoxide, t-amyl hydroperoxide, and cumene hydroperoxide).

These peroxides may be used alone or in combination of two or more. Among them, preferred is an inorganic peroxide, and further preferred is hydrogen peroxide.

The ratio of the peroxide relative to 1 mol of the acid addition salt of the 4-amino-2-halobenzonitrile compound (2) with the organic sulfonic acid is, for example, 0.1 to 10 mol, preferably 1 to 5 mol, and further preferably 1.5 to 2.5 mol. An excessively low ratio of the peroxide may cause a low yield. An excessively high ratio of the peroxide may cause the hydrolysis reaction to further proceed which may produce a carboxylic acid.

The reaction may be carried out in the presence of a solvent. The solvent may be an organic solvent at least containing water as a reaction component.

Examples of the organic solvent may include a polar solvent (such as a protic polar solvent and an aprotic polar solvent).

As examples of the protic polar solvent, there may be mentioned an alcohol (such as a C₁₋₁₂alkyl alcohol such as methanol, ethanol, isopropyl alcohol, hexanol, and decanol).

Examples of the aprotic polar solvent may include an alkyl halide (such as dichloromethane), an amide (such as a chain amide such as N,N-dimethylformamide and N,N-dimethylacetamide, and a cyclic amide such as N-methyl-2-pyrrolidone), a nitrile (such as acetonitrile and propionitrile), a sulfoxide (such as a diC₁₋₆alkyl sulfoxide such as dimethyl sulfoxide), a sulfone (such as a cyclic sulfone such as sulfolane), an ether (such as a chain ether such as diethyl ether and diisopropyl ether, and a cyclic ether such as tetrahydrofuran), a ketone (such as acetone and methyl ethyl ketone), and an ester (such as ethyl acetate).

These organic solvents may be used alone or in combination of two or more. For the use of the combination of two or more, preferred is a mixed solvent of a protic polar solvent and an aprotic polar solvent, further preferred is a mixed solvent of an alcohol and a sulfoxide, and more preferred is a mixed solvent of a C₁₋₃alkyl alcohol and a diC₁₋₃alkyl sulfoxide (such as a mixed solvent of methanol and dimethyl sulfoxide).

The ratio of the organic solvent relative to 100 parts by mass of the acid addition salt of the 4-amino-2-halobenzonitrile compound (2) with the organic sulfonic acid is, for example, 50 to 1000 parts by mass, preferably 100 to 500 parts by mass, further preferably 150 to 300 parts by mass, and more preferably 200 to 250 parts by mass.

The ratio of the protic polar solvent relative to 100 parts by mass of the acid addition salt of the 4-amino-2-halobenzonitrile compound (2) with the organic sulfonic acid is, for example, 10 to 1000 parts by mass, preferably 50 to 800 parts by mass, further preferably 100 to 650 parts by mass, more preferably 150 to 500 parts by mass, and most preferably 180 to 220 parts by mass. An excessively low ratio of the protic polar solvent may cause a low yield. An excessively high ratio of the protic polar solvent may also cause a low yield.

The ratio of the protic polar solvent relative to 100 parts by mass of the aprotic polar solvent is, for example, 200 to 2000 parts by mass, preferably 400 to 1500 parts by mass, further preferably 600 to 1300 parts by mass, and more preferably 800 to 1000 parts by mass. An excessively low ratio of the protic polar solvent may cause a low yield. An excessively high ratio may also cause a low yield.

The ratio of the aprotic polar solvent relative to 100 parts by mass of the acid addition salt of the 4-amino-2-halobenzonitrile compound (2) with the organic sulfonic acid is, for example, 5 to 100 parts by mass, preferably 10 to 80 parts by mass, further preferably 15 to 60 parts by mass, and more preferably 20 to 40 parts by mass.

The ratio of the water relative to 1 mol of the acid addition salt of the 4-amino-2-halobenzonitrile compound (2) with the organic sulfonic acid is preferably 0.1 to 20 mol, further preferably 1 to 15 mol, and more preferably 6 to 10 mol. The ratio of the water relative to 100 parts by mass of the acid addition salt of the 4-amino-2-halobenzonitrile compound (2) with the organic sulfonic acid is, for example, 10 to 100 parts by mass, preferably 20 to 80 parts by mass, further preferably 25 to 60 parts by mass, and more preferably 30 to 50 parts by mass. An excessively low ratio of the water may make the progress of the hydrolysis reaction slow. An excessively high ratio of the water may cause a low yield.

The ratio of the water relative to 100 parts by mass of the total amount of the organic solvent is, for example, 1 to 200 parts by mass, preferably 5 to 100 parts by mass, further preferably 8 to 50 parts by mass, and more preferably 10 to 20 parts by mass. An excessively low ratio of the water may make the progress of the hydrolysis reaction slow. An excessively high ratio of the water may cause a low yield.

The water may be contained in the form of an aqueous solution of the peroxide and/or the base, or may be contained as a mixed solvent of the water and the organic solvent.

The reaction temperature is, for example, 10 to 100°C, preferably 20 to 80°C, further preferably 30 to 60°C, and more preferably 40 to 50°C.

The reaction time is not particularly limited to a specific time and is, for example, 1 to 10 hours, preferably 1.5 to 5 hours, and further preferably 2 to 3 hours.

The reaction may be carried out in air or in an inert gas (such as nitrogen; and a rare or noble gas such as argon and helium). The reaction may be carried out under a normal or ordinary pressure or under an applied pressure. After the completion of the reaction, the reaction product may be separated with purification by a conventional separation-purification means such as neutralization, washing, extraction or elution, concentration, filtration, reprecipitation, centrifugation, precipitation or crystallization, and column chromatography, or a combination of these means. By neutralization or others, the 4-amino-2-halobenzamide compound (6) in the form of the organic sulfonic acid salt may be obtained as a free 4-amino-2-halobenzamide compound (6).

The process for producing the acid addition salt of the 4-amino-2-halobenzonitrile compound (2) with the organic sulfonic acid according to the present invention comprises at least the separation step, and further may comprise the salt formation step as a preceding step as the separation step and/or may comprise the amination step as a preceding step of the salt formation step.

### EXAMPLES

The following examples are intended to describe this invention in further detail and should by no means be interpreted as defining the scope of the invention. The evaluation methods are shown below.

### [¹H-NMR]

The ¹H-NMR was measured using "JNM-ECZ400 (400 MHz)" manufactured by JEOL Ltd. or "JNM-ECA600 (600 MHz)" manufactured by JEOL Ltd., and using chloroform-d (CDCl₃) as a deuterated solvent and tetramethylsilane (TMS) as a standard substance.

### [HPLC]

A sample was dissolved in a mixed solvent of a 0.1% by mass formic acid water and a methanol solution containing 15% by volume acetonitrile (volume ratio: 2/3), and the HPLC area was measured using "High-Pressure Gradient HPLC System/Detector SPD-M20A" manufactured by SHIMADZU CORPORATION. The composition ratio (molar ratio or mole ratio) was calculated from the ratio of the HPLC area percentage. Based on the composition ratio, the purity of the sample can be calculated.

### [Melting point]

The melting point was measured using a melting point measuring apparatus ("535" manufactured by BUCHI) in accordance with Japanese Industrial Standards (JIS) K 4101 (1993) [5.1 Method according to Visual Observation].

### [Synthesis Example 1]

### Synthesis of crude 4-amino-2-fluorobenzonitrile [mixture of 4A2FBN (4-amino-2-fluorobenzonitrile) and 2A4FBN (2-amino-4-fluorobenzonitrile)]

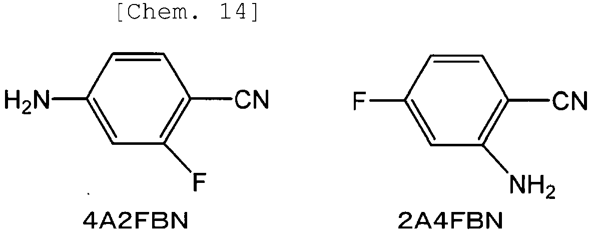

In a 2 L autoclave made of SUS, 242.6 g of dimethyl sulfoxide, 112.3 g (0.8087 mol) of 2,4-difluorobenzonitrile (DFBN), and 245.9 g (5.0 molar ratio (to DFBN)) of 28% by mass ammonia water were charged, and the mixture was heated to 100°C and was subjected to a reaction for 18 hours.

After the completion of the reaction, 587.7 g of the resulting reaction liquid, water, and ethyl acetate were charged in a 1 L flask under a nitrogen atmosphere, and the mixture was stirred and was subjected to liquid-liquid extraction. As an organic layer, 315.7 g of a yellow solution containing crude 4A2FBN and ethyl acetate (a crude 4A2FBN-ethyl acetate solution) was obtained (composition ratio: 4A2FBN/2A4FBN = 58/42, 4A2FBN yield: 57.6% of DFBN).

### [Example 1]

### Purification of crude 4A2FBN (use of p-toluenesulfonic acid as organic sulfonic acid, use of isopropyl alcohol as reaction solvent and precipitation solvent)

In a 500 mL flask, 315.7 g of the crude 4A2FBN-ethyl acetate solution obtained in Synthesis Example 1 was charged and was concentrated under a reduced pressure for solvent removal, and 272.5 g of isopropyl alcohol and 153.8 g (1.0 molar ratio (to DFBN)) of p-toluenesulfonic acid monohydrate were charged in the flask, and then the resulting mixture was subjected to a reaction at 20 to 30°C for one hour.

After the completion of the reaction, the resulting solution was cooled from 28°C to 5°C at a cooling rate of about 0.8°C/min. over about 30 minutes. The precipitated solid was separated by filtration, the separated solid was washed with ethyl acetate, and the washed product was dried under a reduced pressure to obtain 170.9 g of a white solid containing a salt of 4A2FBN with p-toluenesulfonic acid (composition ratio: 4A2FBN/2A4FBN = 99/1, 4A2FBN yield: 53.4% of DFBN).
¹H-NMR (DMSO-d₆): δ (ppm) 2.29 (s, 3H), 6.41-6.46 (m, 2H), 7.13 (d, J=7.6, 2H), 7.38-7.42 (m, 1H), 7.49 (d, J=8.0, 2H), 8.56 (br s, 3H)
Melting point: 219°C

### [Example 2]

Purification of crude 4A2FBN (use of p-toluenesulfonic acid as organic sulfonic acid, use of methanol as reaction solvent and precipitation solvent)

In a 500 mL flask, 315.7 g of the crude 4A2FBN-ethyl acetate solution obtained in Synthesis Example 1 was charged and was concentrated under a reduced pressure for solvent removal, and 272.5 g of methanol and 153.8 g (1.0 molar ratio (to DFBN)) of p-toluenesulfonic acid monohydrate were charged in the flask, and then the resulting mixture was subjected to a reaction at 20 to 30°C for one hour.

After the completion of the reaction, the resulting solution was cooled from 28°C to 5°C at a cooling rate of about 0.8°C/min. over about 30 minutes. The precipitated solid was separated by filtration, the separated solid was washed with methanol, and the washed product was dried under a reduced pressure to obtain 145.3 g of a white solid containing a salt of 4A2FBN with p-toluenesulfonic acid (composition ratio: 4A2FBN/2A4FBN = 98/2, 4A2FBN yield: 45.4% of DFBN).
Melting point: 219°C

### [Example 3]

Purification of crude 4A2FBN (use of p-toluenesulfonic acid as organic sulfonic acid, use of ethyl acetate as reaction solvent and precipitation solvent)

In a 500 mL flask, 315.7 g of the crude 4A2FBN-ethyl acetate solution obtained in Synthesis Example 1 and 153.8 g (1.0 molar ratio (to DFBN)) of p-toluenesulfonic acid monohydrate were charged, and then the resulting mixture was subjected to a reaction at 20 to 30°C for one hour.

After the completion of the reaction, the resulting solution was cooled from 28°C to 5°C at a cooling rate of about 0.8°C/min. over about 30 minutes. The precipitated solid was separated by filtration, the separated solid was washed with ethyl acetate, and the washed product was dried under a reduced pressure to obtain 162.65 g of a white solid containing a salt of 4A2FBN with p-toluenesulfonic acid (composition ratio: 4A2FBN/2A4FBN = 91/9, 4A2FBN yield: 50.8% of DFBN).
Melting point: 219°C

### [Example 4]

Purification of crude 4A2FBN (use of 2-naphthalenesulfonic acid as organic sulfonic acid, use of isopropyl alcohol as reaction solvent and precipitation solvent)

In a 500 mL flask, 315.7 g of the crude 4A2FBN-ethyl acetate solution obtained in Synthesis Example 1 was charged and was concentrated under a reduced pressure for solvent removal, and 272.5 g of isopropyl alcohol and 168.4 g (1.0 molar ratio (to DFBN)) of 2-naphthalenesulfonic acid were charged in the flask, and then the resulting mixture was subjected to a reaction at 20 to 30°C for one hour.

After the completion of the reaction, the resulting solution was cooled from 28°C to 5°C at a cooling rate of about 0.8°C/min. over about 30 minutes. The precipitated solid was separated by filtration, the separated solid was washed with isopropyl alcohol, and the washed product was dried under a reduced pressure to obtain 167.5 g of a white solid containing a salt of 4A2FBN with 2-naphthalenesulfonic acid (composition ratio: 4A2FBN/2A4FBN = 99/1, 4A2FBN yield: 52.3% of DFBN).

### [Example 5]

Purification of crude 4A2FBN (use of 1,5-naphthalenedisulfonic acid as organic sulfonic acid, use of isopropyl alcohol as reaction solvent and precipitation solvent)

In a 500 mL flask, 315.7 g of the obtained crude 4A2FBN-ethyl acetate solution was charged and was concentrated under a reduced pressure for solvent removal, and 272.5 g of isopropyl alcohol and 145.7 g (0.5 molar ratio (to DFBN)) of 1,5-naphthalenedisulfonic acid were charged in the flask, and then the resulting mixture was subjected to a reaction at 20 to 30°C for one hour.

After the completion of the reaction, the resulting solution was cooled from 28°C to 5°C at a cooling rate of about 0.8°C/min. over about 30 minutes. The precipitated solid was separated by filtration, the separated solid was washed with isopropyl alcohol, and the washed product was dried under a reduced pressure to obtain 169.5 g of a white solid containing a salt of 4A2FBN with 1,5-naphthalenedisulfonic acid (composition ratio: 4A2FBN/2A4FBN = 77/23, 4A2FBN yield: 52.9% of DFBN).

### [Example 6]

### Synthesis of 4A2FBAD (4-amino-2-fluorobenzamide)

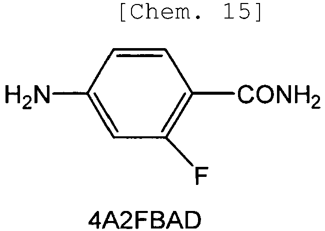

In 2 L flask, 170.9 g (0.4319 mol) of the acid addition salt of 4A2FBN with p-toluenesulfonic acid obtained in Example 1, 336.0 g of methanol, 41.0 g of dimethyl sulfoxide, and 77.0 g (1.25 molar ratio (to the acid addition salt)) of a 28% by mass sodium hydroxide aqueous solution were charged under a nitrogen atmosphere, and then the mixture was heated to 40 to 45°C. To the reaction vessel was added dropwise 83.9 g (2.00 molar ratio (to the acid addition salt)) of a 35% by mass hydrogen peroxide solution. After the completion of the dropwise addition, the resulting mixture was subjected to a reaction at 40 to 45°C for 3 hours to obtain 647.2 g of a reaction liquid. The resulting reaction liquid was adjusted to a pH value of 7 with 36% by mass hydrochloric acid and was subjected to desolvation at 50°C under a reduced pressure to obtain 220.3 g of a concentrated liquid. Into the resulting concentrated liquid, 427.6 g of water was poured, and the resulting mixture was cooled to 0 to 5°C and was stirred for one hour. The produced solid was separated by filtration, and then the separated solid was washed with water, and the washed product was dried to obtain 47.1 g of a light brown solid containing 4A2FBAD (purity by HPLC: 99.0%, theoretical yield: 70.0% of the acid addition salt, consistent yield: 37.4% of DFBN).
Melting point: 148°C

### [Reference Example 1]

Purification of crude 4A2FBN (use of benzoic acid as acid, use of ethyl acetate as reaction solvent and precipitation solvent)

In a 500 mL flask, 315.7 g of the crude 4A2FBN-ethyl acetate solution obtained in Synthesis Example 1 and 98.8 g (1.0 molar ratio (to DFBN)) of benzoic acid were charged, and then the resulting mixture was subjected to a reaction at 20 to 30°C for one hour.

After the completion of the reaction, although the resulting solution was cooled from 28°C to 5°C at a cooling rate of about 0.8°C/min. over about 30 minutes, no solid precipitated. Thus, a benzoic acid salt of 4A2FBN and a benzoic acid salt of 2A4FBN could not be separated.

### [Reference Example 2]

Purification of crude 4A2FBN (use of hydrochloric acid as acid, use of ethyl acetate as reaction solvent and precipitation solvent)

In a 500 mL flask, 315.7 g of the crude 4A2FBN-ethyl acetate solution obtained in Synthesis Example 1 and 82.0 g (1.0 molar ratio (to DFBN)) of 36% by mass hydrochloric acid were charged, and then the resulting mixture was subjected to a reaction at 20 to 30°C for one hour.

After the completion of the reaction, although the resulting solution was cooled from 28°C to 5°C at a cooling rate of about 0.8°C/min. over about 30 minutes, no solid precipitated. Thus, a hydrochloric acid salt of 4A2FBN and a hydrochloric acid salt of 2A4FBN could not be separated.

### [Reference Example 3]

Purification of crude 4A2FBN (use of sulfuric acid as acid, use of isopropyl alcohol as reaction solvent and precipitation solvent)

In a 500 mL flask, 315.7 g of the crude 4A2FBN-ethyl acetate solution obtained in Synthesis Example 1 was charged and was concentrated under a reduced pressure for solvent removal, and then 272.5 g of isopropyl alcohol and 158.7 g (1.0 molar ratio (to DFBN)) of 50% by mass sulfuric acid were charged in the flask, and the resulting mixture was subjected to a reaction at 20 to 30°C for one hour.

After the completion of the reaction, the solution was cooled from 28°C to 5°C at a cooling rate of about 0.8°C/min. over about 30 minutes. The precipitated solid was separated by filtration, the separated solid was washed with isopropyl alcohol, and the washed product was dried under a reduced pressure to obtain 90.4 g of a white solid containing a sulfuric acid salt of 4A2FBN (composition ratio: 4A2FBN/2A4FBN = 53/47, 4A2FBN yield: 28.2% of DFBN).

### [Reference Example 4]

Purification (sublimation purification) of crude 4A2FBN

In a 500 mL flask, 315.7 g of the crude 4A2FBN-ethyl acetate solution obtained in Synthesis Example 1 was charged and was concentrated under a reduced pressure for solvent removal, and then an attempt was made to purify the resulting concentrate by sublimation of an impurity 2A4FBN (isomer) under the conditions of a temperature of 105°C and a pressure (gauge pressure) of -100 kPa. However, the apparatus was clogged with a crystal sublimed within the apparatus and resulted in being inoperable.

### [Reference Example 5]

Purification of crude 4A2FBN (use of toluene as precipitation solvent)

In a 1000 mL flask, 315.7 g of the crude 4A2FBN-ethyl acetate solution obtained in Synthesis Example 1 was charged and was concentrated under a reduced pressure for solvent removal, and 380.8 g of toluene was charged in the flask. The resulting mixture was stirred at 20 to 30°C for one hour and then was allowed to stand.

Thereafter, the precipitated solid was separated by filtration, the separated solid was washed with ethyl acetate, and the washed product was dried under a reduced pressure to obtain 24.2 g of a white solid containing 4A2FBN (composition ratio: 4A2FBN/2A4FBN = 99/1, 4A2FBN yield: 21.6% of DFBN).
Melting point: 56°C

### INDUSTRIAL APPLICABILITY

The acid addition salt obtained in the production process of the present invention can be used as a raw material for a pharmaceutical intermediate, a reagent, and the like.

## Claims

1. A process for producing an acid addition salt of a 4-amino-2-halobenzonitrile compound with an organic sulfonic acid, the process comprising a separation step of separating
an acid addition salt of a 4-amino-2-halobenzonitrile compound represented by the following formula (2) with an organic sulfonic acid
from an acid addition salt of an isomeric mixture of an aminohalobenzonitrile compound represented by the following formula (1) with an organic sulfonic acid
using a difference in solubility between acid addition salts of isomers in a solvent: 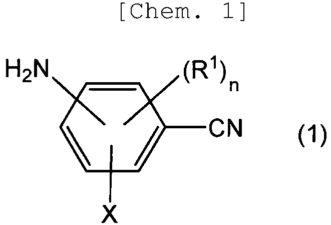
wherein
X represents a halogen atom,
R¹ represents a reactively inactive substituent, and
n denotes an integer of 0 to 3, 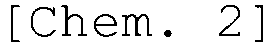 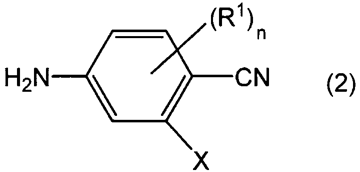
wherein
X, R¹, and n each have the same meanings as those in the formula (1).

2. The process according to claim 1, wherein, in the separation step, the separation using the difference in solubility between acid addition salts of isomers in the solvent includes precipitation and/or extraction.

3. The process according to claim 1 or 2, wherein, in the separation step, the solvent contains an ester and/or an alcohol.

4. The process according to claim 1, which comprises a salt formation step of allowing the isomeric mixture of the aminohalobenzonitrile compound represented by the formula (1) at least containing the 4-amino-2-halobenzonitrile compound represented by the formula (2) to react with the organic sulfonic acid to form an acid addition salt.

5. The process according to claim 4, which comprises, as a preceding step of the salt formation step, an amination step of subjecting a dihalobenzonitrile compound represented by the following formula (4) at least containing a 2,4-dihalobenzonitrile compound represented by the following formula (3) to an amination reaction: 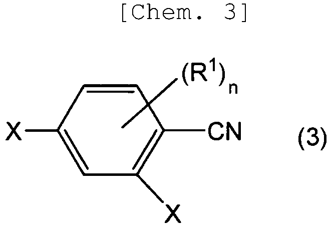 wherein
each X independently represents a halogen atom, and
R¹ and n each have the same meanings as those in the formula (1), 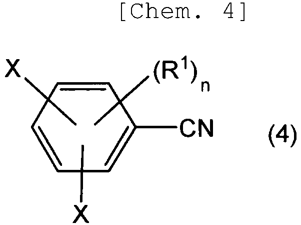
wherein
X, R¹, and n each have the same meanings as those in the formula (3).

6. The process according to claim 4, wherein the reaction in the salt formation step is carried out in the presence of a solvent, and the solvent used in the salt formation step is the same as the solvent used in the separation step.

7. The process according to any one of claims 1, 2, and 4 to 6, wherein the organic sulfonic acid contains an aromatic sulfonic acid.

8. The process according to claim 7, wherein the aromatic sulfonic acid contains an arene ring-containing sulfonic acid represented by the following formula (5): 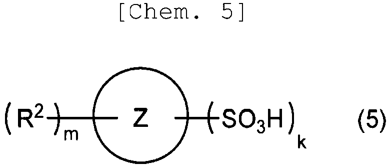
wherein
a ring Z represents an arene ring,
R² represents a reactively inactive substituent,
m denotes an integer of not less than 0, and
k denotes an integer of not less than 1.

9. The process according to claim 8, wherein, in the formula (5), the ring Z represents a C₆₋₁₄arene ring, R² represents a straight- or branched-chain C₁₋₃alkyl group, m denotes 0 or 1, and k denotes 1 or 2.

10. The process according to any one of claims 1, 2, and 4 to 6, wherein a ratio of the sulfonic acid group of the organic sulfonic acid is 0.8 to 1.2 mol relative to 1 mol of a total amount of the isomeric mixture of the aminohalobenzonitrile compound represented by the formula (1).

11. A process for producing a 4-amino-2-halobenzamide compound represented by the following formula (6) or an acid addition salt thereof: 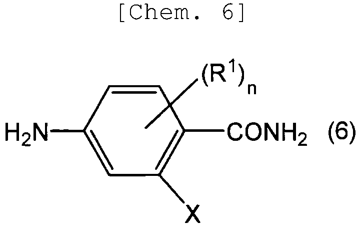 wherein
X, R¹, and n each have the same meanings as those in the formula (1) recited in claim 1,
the process comprising an amidation step of subjecting an acid addition salt produced by a process recited in any one of claims 1 to 6 to a hydrolysis reaction.

12. An acid addition salt of a 4-amino-2-halobenzonitrile compound represented by the following formula (2) with an organic sulfonic acid: 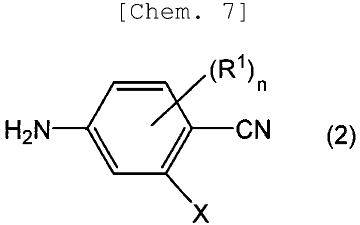 wherein
X represents a halogen atom,
R¹ represents a reactively inactive substituent, and
n denotes an integer of 0 to 3.
